# NEW EUROPEAN PATENT SPECIFICATION

(11) **EP 1 458 321 B2**
(45) Date of publication and mention of the opposition decision: **26.12.2012**
(45) Mention of the grant of the patent: 14.04.2010
(21) Application number: 02761576.4
(22) Date of filing: 05.09.2002
(51) Int. Cl.: A61F 13/15, A61F 13/49

(54) **METHOD FOR MAKING AN ABSORBENT ARTICLE WITH ELASTIC CUFF AREAS AND NECKED SUBSTRATES**
VERFAHREN ZUM HERSTELLEN VON ABSORBIERENDEN ARTIKELN MIT ELASTISCHEN RANDBEREICHEN UND EINGESCHNÜRTEN FLÄCHENGEBILDEN
PROCEDE DE PRODUCTION D'UN ARTICLE ABSORBANT DOTE DE ZONES REVERS ELASTIQUES ET DE SUBSTRATS ETIRES

(30) Priority: 19.12.2001 US 25027
(43) Date of publication of application: 22.09.2004
(73) Proprietor: KIMBERLY-CLARK WORLDWIDE, INC., Neenah, WI 54956 (US)
(72) Inventor: MORMAN, Michael, Tod, Alpharetta, GA 30022 (US); ROESSLER, Thomas, Harold, Appleton, Wisconsin 54913 (US)
(74) Representative: Mabey, Katherine Frances
(86) International application number: PCT/US2002/028272
(87) International publication number: WO 2003/053317

(56) References cited:
- WO-A-00/38911
- WO-A-01/15646
- WO-A-96/10481
- US-A- 5 156 793
- US-A- 5 226 992
- US-A- 5 503 919
- US-A- 5 680 653
- US-A- 5 846 232
- US-A- 5 910 224

## Description

### BACKGROUND OF THE INVENTION

### Field of the Invention

It is desired that absorbent articles, and especially garments such as diapers, training pants or incontinence garments, without limitation referred to generically now for ease of explanation as "diapers", provide a close, comfortable fit about the waist of the wearer and contain body exudates while maintaining skin health. In certain circumstances, it is also desirable that such garments are capable of being pulled up or down over the hips of the wearer to allow the wearer or caregiver to easily pull the article on and easily remove the article. Other garment openings such as sleeve or pant cuffs and necklines may benefit from similar elasticizing.

Various schemes for producing elastic waistbands on disposable diapers have been proposed. Diaper waistbands are generally made by stretching an elastomer, applying the stretched elastomer to the diaper components, typically non-elastic in the waistband area, and allowing the elastomer to retract, thus gathering and shirring the attached diaper web components in the waistband area. The gathered waistband will then ungather to some extent when applied to a wearer, to give the waistband circumference some extension while the elastomer produces a retractive force holding the waistband snug to the wearer. However, this gathered-material waistband may be esthetically unappealing as looking less like a fully finished cloth garment or functionally deficient as forming channels for the leakage of exudates. Further, manipulation and application of the stretched, or tensioned, elastics to nonwoven webs moving at high speeds is difficult.

Thus, there remains a need for other methods of making waistbands or other elasticized openings for disposable garments which provide ease and economy of manufacture, and adequate performance. The present invention provides a method of producing selectively elastic areas in a web suitable for garments.

In response to the above discussion, an alternative method of elastomeric cuff construction is provided by the present invention which provides improved ease and economy of manufacture, while maintaining adequate fit and improved sealing performance.

The methods of the present invention allow elastomeric materials to be incorporated into the diaper assembly in a simplified fashion. In particular, with presently known methods, elastomeric materials are stretched, cut and held under tension. This type of manipulation of individual pieces of unstable elastomeric materials makes incorporation into a diaper assembly challenging. With the methods of the present invention, the elastomeric materials do not have to be under tension or can be under less tension than is necessary with conventional methods. This makes manipulation of the elastomeric materials easier. For example, the elastomeric materials that are elastomeric in the cross-direction can be applied without rotation.

The methods of the invention may involve the application of elastomeric materials in either an untensioned state and a reduced tensioned state, or both. The selection of the tension under which the elastomeric materials are applied depends on how best to insure that the waistband will be flat in use so as to form a good seal. It is desirable to have a small circumference of the product at the waist for purposes of forming a good seal with the wearer's body and for purposes of eliminating any gathers or rugosities that could serve as channels for the leakage of body exudates. For example, when manufacturing a diaper to be used on small babies, it may be desirable to apply the elastomeric materials under low tension so that the finished diaper has some gathered material in the waistband. However, when such a diaper is applied to a baby, the waistband is extended by the baby's body, the gathers are removed and the result is a flat waistband against the baby's body during wear. While the desirability of a flat waistband is described, it is also desirable, for the same reasons, to be able to provide flat leg elastics.

The methods of the invention permit the use of non-composite elastomerics (e.g. films, meltblown, etc.) in addition to conventional elastomeric materials that require nonwoven facings.

The present invention presents an alternative way of making a elastomeric cuff area, such as a waistband, by temporarily narrowing, or necking, one dimension of one or more of the diaper components of the precursor garment, or garment webs, and attaching an elastomer, whether stretched or unstretched, to the cuff area of the necked precursor garment web. When the narrowing force, which may be applied in any direction necessary to neck the garment components, is removed, the necked garment component or components will relax and expand, or may be physically caused to expand, except where the elastomer was attached, thus forming a desirable narrowed, and substantially flat, elastomeric cuff area having expandable dimension and elastic tension, and providing an efficient seal against the skin of the wearer. In other embodiments, garment openings such as sleeve or leg cuffs, or necklines, may benefit from similar elasticizing. The margins of any garment opening can be collectively referred to as "cuffs" or "cuff areas".

The person having ordinary skill in the art of disposable diaper manufacture will appreciate that the disposable diaper is generally made up of the layers of a substantially liquid-impermeable backsheet or outer cover, a liquid-permeable topsheet or bodyside liner and an liquid retention or absorbent structure located between the backsheet and the topsheet. In order to be made extendible, such as by necking, any two joined layers must have compatible stretch to the limits of the desired processing parameters. In other words, the combined layers or webs, in those areas where the webs are fastened together, will be limited in the amount they may be stretched by the properties of the layer having the least amount of stretch.

Generally, the method of the present invention may produce a disposable absorbent article such as a diaper, although the skilled artisan will appreciate that the teachings herein may be applied to any type garment with a need for elastomeric cuffs. The diaper defines a front waist section, a rear waist section, an intermediate section which extends between and connects the waist sections, a pair of laterally opposed side edges, a pair of longitudinally opposed waist edges, a longitudinal direction and a lateral direction. Exemplary elastic waistbands for waist sections are provided in a unique fashion with elasticity to help the waist sections conform to the body of the wearer.

The absorbent article may also include other known components of diapers such as a pair of fasteners located on the laterally opposed side edges in one of the waist sections. In certain aspects, the disposable absorbent article may be provided in a prefastened, pant-like configuration such that the article can be pulled on or off over the hips of the wearer similar to conventional training pants. For example, the fasteners may refastenably attach the laterally opposed side edges in the front waist section to the laterally opposed side edges in the rear waist section to provide the pant-like, prefastened absorbent article prior to packaging the articles.

There are various ways to accomplish the present invention.

For example, the diaper backsheet, or outer cover; and topsheet, or bodyside liner; or both, may be necked as separate webs, or joined webs, during assembly into the precursor diaper in the diaper making process i.e., converting the components into a garment. A necked backsheet laminate according to one aspect of the present invention will generally remain flat in its extended position. An untensed elastomeric may then be applied to the necked and flat area resulting in an ungathered elasticized waistband or leg elastic area providing an efficacious and comfortable seal area against the body of the wearer.

Alternatively, the diaper assembly may be produced with all but the elastomeric cuffs and then be longitudinally stretched, laterally stretched, or both, prior to applying the cuff elastics. This stretching can cause the entire diaper assembly to try to neck. Elastomer is then applied to the desired cuff area. A waistband or leg cuff area of reduced dimension is formed after the stretching or tensioning, force is removed and the elastomeric holds the reduced dimension area at the narrower dimension while the remaining area of the diaper retains or regains its wider dimension.

In another alternative, an unstretched elastomer can be applied to the regular diaper assembly and the assembly with the elastomer thereon stretched to neck a cuff area. Heating the elastomer to a high enough temperature while it is necked will cause the elastomer to set at its narrowed width to form the flat cuff area. This method and the second method above could also be used with a thermoset elastomer where the elastomeric pre-cursor is placed on the necked, or to-be-necked, waistband area and then cross-linked. These three processes might be used individually or in combination with each other or with the known pre-stretched elastomer process to make excellent waistbands.

While an exemplary embodiment of the invention is shown with the precursor garments oriented in the machine direction of the webs, it will be appreciated that the techniques of the present invention may be adapted to situations where the longitudinal direction of the precursor garments is in the cross machine direction.

### BRIEF DESCRIPTION OF THE DRAWINGS

The present invention will be more fully understood and further advantages will become apparent when reference is made to the following detailed description of the invention and the drawings, in which:
Fig. 1 representatively shows a partially cut-away, top plan view of the inward surface of an example of an article;
Fig. 2 illustrates a beginning manufacturing sequence of disposable diapers according to the present invention with the longitudinal direction of the diapers being in the Machine Direction for ease of necking the desired material webs.
Fig. 3 illustrates a middle manufacturing sequence of disposable diapers according to the present invention with the longitudinal direction of the diapers being in the Machine Direction for ease of necking the desired material webs.
Fig. 4 illustrates an ending manufacturing sequence including the individuation of disposable diapers from the precursor garment assemblage according to the present invention with the longitudinal direction of the diapers being in the Machine Direction for ease of necking the desired material webs.
Fig. 5 illustrates an alternative embodiment of the invention wherein the precursor garment is completely assembled except for the waist elastics before the waistband areas of the garment are necked.
Fig. 6 illustrates an alternative embodiment of the invention wherein a precursor elastomer is applied to at least one layer of the precursor garment before the layer is necked and the precursor elastic is then treated while the layer is necked in order to turn the precursor elastic into an elastomeric material.

### DEFINITIONS

As used herein, the term "nonwoven web" means a web of fibrous material that is formed without the aid of a textile weaving or knitting process. The term "fabrics" is used to refer to all of the woven, knitted and nonwoven fibrous webs.

As used herein, the terms "neck" or "neck stretch" and forms thereof, interchangeably mean that the fabric is drawn such that it is extended under conditions reducing its width or its transverse dimension by drawing and elongating to increase the length of the fabric. The controlled drawing may take place under cool temperatures, room temperature or greater temperatures and is limited to an increase in overall dimension in the direction being drawn up to the elongation required to break the fabric. The necking process typically involves unwinding a sheet from a supply roll and passing it through a brake nip roll assembly driven at a given linear speed. A take-up roll or nip, operating at a linear speed higher than the brake nip roll, draws the fabric and generates the tension needed to elongate and neck the fabric. U.S. Patent No. 4,965,122, issued October 23, 1990, to Morman, discloses a process for providing a reversibly necked nonwoven material which may include necking the material, then heating the necked material, followed by cooling.

As used herein, the term "neckable material or layer" means any material which can be necked such as a nonwoven, woven, or knitted material. As used herein, the term "necked material" refers to any material which has been drawn in at least one dimension (e.g. lengthwise) reducing the transverse dimension, (e.g. width), such that when the drawing force is removed, the material can be pulled back, or relax, to, or near, its original width. The necked material typically has a higher basis weight per unit area than the un-necked material. When the necked material returns to its original un-necked width, it should have about the same basis weight as the un-necked material. This differs from stretching/orienting a material layer, such as a film, during which the layer is thinned and the basis weight is permanently reduced.

Typically, such necked nonwoven fabric materials are capable of being necked up to about 80 percent (or to about 20 percent of their original width). For example, the neckable backsheet 30 of the various aspects of the present invention may be provided by a material that has been necked from about 10 to about 80 percent, desirably from about 20 to about 60 percent, and more desirably from about 30 to about 50 percent for improved performance. For the purposes of the present disclosure, the term "percent necked" or "percent neckdown" refers to a ratio or percentage determined by measuring the difference between the pre-necked dimension and the necked dimension of a neckable material, and then dividing that difference by the pre-necked dimension of the neckable material and multiplying by 100 for percentage. The percentage of necking (percent neck) can be determined in accordance with the description in the above-mentioned U.S. Patent No. 4,965,122.

As used herein, the terms "elastic", "elastomeric", and forms thereof, mean any material which, in its final form in the completed diaper, upon application of a biasing force, is stretchable, that is, elongatable, and which will return to substantially its original shape upon release of the stretching, elongating force. The term will include precursor elastomerics which are heat activated or otherwise subsequently treated after application to the precursor diaper structure to induce elasticity. By the term "non-elastic", what is meant is that the sheet layers are made from polymers that are generally considered to be inelastic. In other words, use of such inelastic polymers to form the sheet layers would result in sheet layers that are not elastic.

As used herein, the term "machine direction", or MD, means the length of a fabric in the direction in which it is produced. The term "cross machine direction" or CD means the width of fabric, i.e. a direction generally perpendicular to the MD.

Words of degree, such as "about", "substantially", and the like are used herein in the sense of "at, or nearly at when given the manufacturing and material tolerances inherent in the stated circumstances" and are used to prevent the unscrupulous infringe from unfairly taking advantage of the invention disclosure where exact figures or absolutes are stated as an aid to understanding the invention.

"Precursor" as used herein means those components, materials, assemblies, or the like which are used or exist in the making of a finished diaper before its completion as a commercially ready product.

"Causing" can include active causation, as for example in actively stretching the web to a second width, and passive causation, as in merely allowing the web to resume a wider width after removing the necking tension.

### DETAILED DESCRIPTION

The various aspects and embodiments of the invention will be described in the context of disposable absorbent articles, and more particularly referred to, without limitation and by way of illustration only, as a disposable diaper with an elastic waistband. It is, however, readily apparent that the present invention could also be employed to produce other elasticized cuff areas and other garments, such as feminine care articles, various incontinence garments, medical garments and any other disposable garments, whether absorbent or not, needing an easily manufactured elasticized area around an opening in the garment. Typically, the disposable garments are intended for limited use and are not intended to be laundered or otherwise cleaned for reuse. A disposable diaper, for example, is discarded after it has become soiled by the wearer.

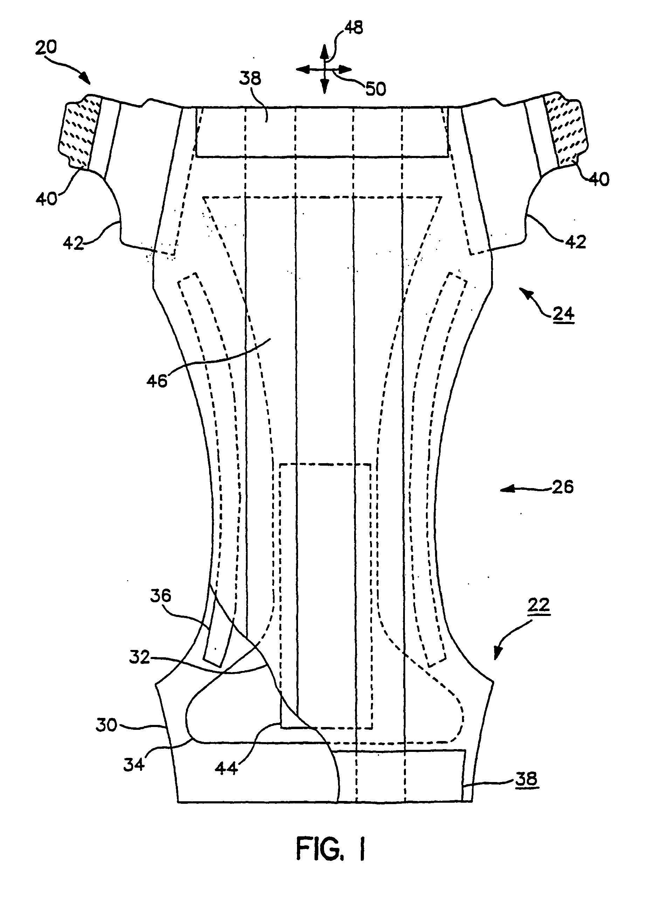

Figure 1 is a representative plan view of an absorbent article, such as disposable diaper 20, in its flat-out, or unfolded state. Portions of the structure are partially cut away to more clearly show the interior construction of diaper 20. The surface of the diaper 20 which contacts the wearer is facing the viewer.

With reference to Fig. 1, the disposable diaper 20 generally defines a front waist section 22, a rear waist section 24, and an intermediate section 26 which interconnects the front and rear waist sections. The front and rear waist sections 22 and 24 include the general portions of the diaper which are constructed to extend substantially over the wearer's front and rear abdominal regions, respectively, during use. The intermediate section 26 of the diaper includes the general portion of the diaper that is constructed to extend through the wearer's crotch region between the legs. Thus, the intermediate section 26 is an area where repeated liquid surges typically occur in the diaper.

The diaper 20 includes, without limitation, an outer cover, or backsheet 30, a liquid permeable bodyside liner, or topsheet, 32 positioned in facing relation with the backsheet 30, and an absorbent body, or liquid retention structure, 34, such as an absorbent pad, which is located between the backsheet 30 and the topsheet 32. The backsheet 30 defines a length, or longitudinal direction 48, and a width, or lateral direction 50 which, in the illustrated embodiment, coincide with the length and width of the diaper 20. The liquid retention structure 34 generally has a length and width that are less than the length and width of the backsheet 30, respectively. Thus, marginal portions of the diaper 20, such as marginal sections of the backsheet 30, may extend past the terminal edges of the liquid retention structure 34. In the illustrated embodiments, for example, the backsheet 30 extends outwardly beyond the terminal marginal edges of the liquid retention structure 34 to form side margins and end margins of the diaper 20. The topsheet 32 is generally coextensive with the backsheet 30 but may optionally cover an area which is larger or smaller than the area of the backsheet 30, as desired.

To provide improved fit and to help reduce leakage of body exudates from the diaper 20, the diaper side margins and end margins may be elasticized with suitable elastic members, as further explained below. For example, as representatively illustrated in Fig. 1, the diaper 20 may include leg elastics 36 which are constructed to operably tension the side margins of the diaper 20 to provide elasticized leg bands which can closely fit around the legs of the wearer to reduce leakage and provide improved comfort and appearance. Waist elastics 38 are employed to elasticize the end margins of the diaper 20 to provide elasticized waistbands. The waist elastics 38 are configured to provide a resilient, comfortably close fit around the waist of the wearer.

Materials suitable for use as the leg elastics 36 and waist elastics 38 are well known to those skilled in the art. Exemplary of such materials are sheets or strands or ribbons of a polymeric, elastomeric material which are adhered to the backsheet, such that elastic constrictive forces are imparted to the backsheet 30. The elastics may also include such materials as polyurethane, synthetic and natural rubber that may optionally be heat shrinkable or heat elasticizable. Many variants of elastomeric materials suitable for use with the present invention will occur to the person having ordinary skill in the art upon gaining an understanding of the invention as presented herein.

As is known, fastening means, such as hook and loop fasteners, may be employed to secure the diaper 20 on a wearer. Alternatively, other fastening means, such as buttons, pins, snaps, adhesive tape fasteners, cohesives, fabric-and-loop fasteners, or the like, may be employed. In the illustrated embodiment, the diaper 20 includes a pair of side panels 42 to which the fasteners 40, indicated as the hook portion of a hook and loop fastener, are attached. Generally, the side panels 42 are attached to the side edges of the diaper 20 in one of the waist sections 22, 24 and extend laterally outward therefrom. The side panels 42 may be elasticized or otherwise rendered elastomeric. For example, the side panels 42, or indeed, any precursor webs of the garment, may be an elastomeric material such as a neck-bonded laminate (NBL) or stretch-bonded laminate (SBL) material. Methods of making such materials are well known to those skilled in the art and are described in U.S. Patent No. 4,663,220 issued May 5, 1987 to Wisneski et al., U.S. Patent No. 5,226,992 issued July 13, 1993 to Morman, and European Patent Application No. EP 0 217 032 published on April 8, 1987 in the names of Taylor et al. Examples of absorbent articles that include elasticized side panels and selectively configured fastener tabs are described in PCT Patent Application No. WO 95/16425 published June 22, 1995 to Roessler; U.S. Patent No. 5,399,219 issued March 21, 1995 to Roessler et al.; U.S. Patent No. 5,540,796 to Fries; and U.S. Patent No. 5,595,618 to Fries.

The diaper 20 may also include a surge management layer 44, located between the topsheet 32 and the liquid retention structure 34, to rapidly except fluid exudates and distribute the fluid exudates to the liquid retention structure 34 within the diaper 20. The diaper 20 may further include a ventilation layer (not illustrated) located between the liquid retention structure 34 and the backsheet 30 to insulate the backsheet 30 from the liquid retention structure 34 to reduce the dampness of the garment at the exterior surface of the backsheet 30. Examples of suitable surge management layers 44 are described in U.S. Patent No. 5,486,166 to Bishop and U.S. Patent No. 5,490,846 to Ellis.

As representatively illustrated in Fig. 1, the disposable diaper 20 may also include a pair of containment flaps 46 which are configured to provide a barrier to the lateral flow of body exudates. The containment flaps 46 may be located along the laterally opposed side edges of the diaper 20 adjacent the side edges of the liquid retention structure 34. Each containment flap 46 typically defines an unattached edge which is configured to maintain an upright, perpendicular configuration in at least the intermediate section 26 of the diaper 20 to form a seal against the wearer's body. The containment flaps 46 may extend longitudinally along the entire length of the liquid retention structure 34 or may only extend partially along the length of the liquid retention structure 34. When the containment flaps 46 are shorter in length than the liquid retention structure 34, the containment flaps 46 can be selectively positioned anywhere along the side edges of the diaper 20 in the intermediate section 26. Such containment flaps 46 are generally well known to those skilled in the art. For example, suitable constructions and arrangements for containment flaps 46 are described in U.S. Patent No. 4,704,96 issued November 3, 1987 to K. Enloe.

The diaper 20 may be of various suitable shapes. For example, the diaper may have an overall rectangular shape, T-shape or an approximately hour-glass shape. In the shown embodiment, the diaper 20 has a generally I-shape. The diaper 20 further has a longitudinal direction 48, and a lateral direction 50. Other suitable components which may be incorporated on absorbent articles of the present invention may include waist flaps and the like which are generally known to those skilled in the art. Examples of diaper configurations suitable for use in connection with the instant invention which may include other components suitable for use on diapers are described in U.S. Patent No. 4,798,603 issued January 17, 1989 to Meyer et al.; U.S. Patent No. 5,176,668 issued January 5, 1993 to Bernardin; U.S. Patent No. 5,176,672 issued January 5, 1993 to Bruemmer et al.; U.S. Patent No. 5,192,606 issued March 9, 1993 to Proxmire et al.; and U.S. Patent No. 5,509,915 issued April 23, 1996 to Hanson et al.

The various components of the diaper 20 are assembled together employing various types of suitable attachment means, such as adhesive, ultrasonic bonds, thermal bonds or combinations thereof. In the shown embodiment, for example, the topsheet 32 and backsheet 30 may be assembled to each other and to the liquid retention structure 34 with lines of adhesive, such as a hot melt, pressure-sensitive adhesive. Similarly, other diaper components, such as the elastic members 36 and 38, fastening members 40, and surge layer 44 may be assembled into the article by employing the above-identified attachment mechanisms.

The illustrated diaper 20 includes a distinctive waistband structure formed by applying an elastomeric 38 to at least the backsheet 30. As known in the art, the backsheet 30 generally includes a fabric or material layer which may be operatively attached or otherwise joined to the other diaper layers to extend over a major portion of the outward surface of the diaper. According to the present invention, the waistband structure is formed by applying an elastomeric to that layer of the diaper which is neckable to a narrowed lateral dimension and which can resume its wider original lateral dimension upon removal of the necking force. At least portions of the backsheet 30 in the waistband area are not affixed to non-neckable portions of the diaper or otherwise restricted from extending and reexpanding. It will occur to the person having ordinary skill in the art that if the backsheet is not used in conjunction with the diaper waistband, other layers used in the construction of a diaper may be similarly utilized according to the precepts of the present invention. Generally, it is desirable for simplicity of construction that the backsheet 30 remains the structural unit of choice for applying the waistband elastomeric in the making of diapers according to the present invention.

Desirably, the neckable backsheet 30 is constructed to be permeable to at least water vapor. For example, in particular embodiments, the neckable backsheet 30 defines a water vapor transmission rate (WVTR) according to the Mocon Water Vapor Transmission Rate Test of at least about 400 g/sq.m/24 hr., desirably at least about 1200 g/sq.m/24 hr, more desirably at least about 2000 g/sq.m/24 hr., and even more desirably at least about 3000 g/sq.m/24 hr. in the non-extended condition. In such embodiments, the neckable backsheet 30 may define a WVTR of from about 400 to about 60,000 g/sq.m/24 hr. Materials which have a WVTR less than those above may not allow a sufficient amount of water vapor diffusion out of the diaper and undesirably result in increased levels of skin hydration. A Mocon WVTR test is described in U.S. Patent 6,156,421 issued December 5, 2000 to Stopper et al.

The backsheet 30 can be composed of any of various materials that provide the desired properties of neckability and lateral extendibility when necking tension is removed. For example, the backsheet 30 can be composed of nonwoven webs such as spunbond fabrics, films, or combinations thereof. In a particular embodiment, the backsheet 30 can be composed of an laminate of two or more layers. For example, the backsheet 30 may be a neckable laminate formed from at least one necked fabric laminated to at least one extendable film material wherein the necked laminate is expandable in at least one direction.

Suitable non-elastic neckable materials for the present invention include nonwoven webs, woven materials and knitted materials such as those described in the above-mentioned U.S. Patent No. 4,965,122. Nonwoven fabrics or webs have been formed from many processes, for example, bonded carded web processes, meltblowing processes and spunbonding processes. The non-elastic neckable material is preferably formed from at least one member selected from fibers and filaments of inelastic polymers. Such polymers include polyesters, for example, polyethylene terephthalate; polyolefins, for example, polyethylene and polypropylene; polyamides, for example, nylon 6 and nylon 66. These fibers or filaments are used alone or in a mixture of two or more thereof. Suitable fibers for forming the neckable material include natural and synthetic fibers as well as bicomponent, multi-component, and shaped polymer fibers. Many polyolefins are available for fiber production according to the present invention, for example, fiber forming polypropylenes include Exxon Chemical Company's Escorene® PD 3445 polypropylene and Himont Chemical Company's PF-304. Polyethylenes such as Dow Chemical's ASPUN® 6811A linear low density polyethylene, 2553 LLDPE and 25355 and 12350 high density polyethylene are also suitable polymers. The nonwoven web layer may be bonded to impart a discrete bond pattern with a prescribed bond surface area. If too much bond area is present on the neckable material, it will break before it necks. If there is not enough bond area, then the neckable material will pull apart. Typically, the percent bonding area useful in the present invention ranges from around five percent to around forty percent of the area of the neckable material. Alternative necked laminate materials that could be used to provide the backsheet 3.0 of the different aspects of the present invention are described in U.S. Patent Application No. 09/460,490 filed December 14, 1999 and entitled "BREATHABLE LAMINATE PERMANENTLY CONFORMABLE TO THE CONTOURS OF A WEARER".

The topsheet 32, as representatively illustrated in Fig. 1, typically presents a body-facing surface that is compliant, soft-feeling, and non-irritating to the wearer's skin. Further, the topsheet 32 can be less hydrophilic than the liquid retention structure 34, and is sufficiently porous to be liquid permeable, permitting liquid to readily penetrate through its thickness to reach the absorbent composite. A suitable topsheet layer 32 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The topsheet layer 32 is typically employed to help isolate the wearer's skin from liquids held in the liquid retention structure 34.

Various woven and nonwoven fabrics can be used for topsheet 32. For example, the topsheet may be composed of a meltblown or spunbond web of the desired fibers, and may also be a bonded-carded-web. Layers of different materials that may have different fiber deniers can also be used. The various fabrics can be composed of natural fibers, synthetic fibers or combinations thereof. The topsheet 32 may be composed of a substantially hydrophobic material, and the hydrophobic material may optionally be treated with a surfactant or otherwise processed to impart a desired level of wettability and hydrophilicity. In a particular embodiment of the invention, topsheet 32 can be a nonwoven, spunbond polypropylene fabric composed of about 2.0 - 5.0 denier fibers formed into a web having a basis weight of about 22 gsm and density of about 0.06 gm/cc. The fabric can be surface treated with an operative amount of surfactant, as known in the art. The surfactant can be applied by any conventional means, such as spraying, printing, brush coating or the like.

The topsheet 32 may be made from any suitable neckable materials compatible with the backsheet 30. Suitable materials for use with the present invention may include nonwoven webs, woven materials and knitted materials. Such webs can include one or more fabric layers. Nonwoven fabrics or webs have been formed from many processes, for example, bonded carded web processes, meltblowing processes and spunbonding processes. For example, a non-elastic neckable material may be formed from at least one member selected from fibers and filaments of inelastic polymers. Such polymers include polyesters, for example, polyethylene terephthalate, polyolefins, for example, polyethylene and polypropylene, polyamides, for example, nylon 6 and nylon 66. These fibers or filaments are used alone or in a mixture of two or more thereof. Suitable fibers for forming the neckable material include natural and synthetic fibers as well as bicomponent, multi-component, and shaped polymer fibers. Many polyolefins are available for fiber production according to the present invention, for example, fiber forming polypropylenes include Exxon Chemical Company's Escorene® PD 3445 polypropylene and Himont Chemical Company's PF-304. Polyethylenes such as Dow Chemical's ASPUN® 6811A linear low density polyethylene, 2553 LLDPE and 25355 and 12350 high density polyethylene are also suitable polymers. The nonwoven web layer may be bonded to impart a discrete bond pattern with a prescribed bond surface area. If too much bond area is present on the neckable material, it will break before it necks. If there is not enough bond area, then the neckable material will pull apart. Typically, the percent bonding area useful in the present invention ranges from around 5 percent to around 40 percent of the area of the neckable material. For example, a particularly suitable material for the topsheet 32 is a necked spunbond web of polypropylene fibers having a basis weight of from about 5 to about 30 gsm. Such a web may be necked up to about 80 percent.

The neckable material may be necked to form a narrowed waistband area by conventional necking processes that typically vary the surface speed of the web to draw or neck the material. Such necking will cause the material to retract and permit the material to reexpand in the lateral direction when necking tension is removed. Such necked nonwoven fabric materials typically are capable of being necked up to about 80 percent. For example, the backsheet 30, or topsheet 32, in various aspects of the present invention may be necked from about 10 to about 80 percent, desirably from about 20 to about 60 percent, and more desirably from about 30 to about 50 percent for improved performance.

The topsheet 32 and backsheet 30 may be connected or otherwise associated together in an operable manner. As used herein, the term "associated" encompasses configurations in which topsheet 32 is directly joined to the backsheet 30 by affixing the topsheet 32 directly to the backsheet 30, and configurations wherein the topsheet 32 is indirectly joined to the backsheet 30 by affixing the topsheet 32 to intermediate members which in turn are affixed to the backsheet 30. The topsheet 32 and the backsheet 30 can, for example, be joined to each other in at least a portion of the diaper periphery by attachment mechanisms (not shown) such as adhesive bonds, sonic bonds, thermal bonds, pinning, stitching or any other attachment techniques known in the art, as well as combinations thereof.

For example, a uniform continuous layer of adhesive, a patterned layer of adhesive, a sprayed pattern of adhesive or an array of separate lines, swirls or spots of construction bonds may be used to affix the topsheet 32 to the backsheet 30. It should be readily appreciated that the above-described attachment mechanisms may also be employed to suitably interconnect, assemble and/or affix together the various other component parts of the garments or articles that are described herein.

The liquid retention structure 34 provides an absorbent structure for holding and storing absorbed liquids and other waste materials, such as the shown absorbent pad composed of selected hydrophilic fibers and high-absorbency particles. The liquid retention structure 34 may also be neckable, not neckable, or elastic, although it should not interfere with the necking of the waistband area. The liquid retention structure 34 is positioned and sandwiched between the topsheet 32 and backsheet 30 to form the diaper 20. The liquid retention structure 34 has a construction that is generally compressible, conformable, non-irritating to the wearer's skin, and capable of absorbing and retaining body exudates. It should be understood that, for purposes of this invention, the liquid retention structure may be a single, integral piece of material, or alternatively, may have a plurality of individual separate pieces of material which are operably assembled together.

Various types of wettable, hydrophilic fibrous material can be used to form the component parts of liquid retention structure 34. Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers; and synthetic fibers composed of a nonwettable thermoplastic polymer, such as polypropylene fibers, which have been hydrophilized by appropriate means. The fibers may be hydrophilized, for example, by treatment with silica, treatment with a material which has a suitable hydrophilic moiety and is not readily removable from the fiber, or by sheathing the nonwettable, hydrophobic fiber with a hydrophilic polymer during or after the formation of the fiber. For the purposes of the present invention, it is contemplated that selected blends of the various types of fibers mentioned above may also be employed. The liquid retention structure 34 can include a matrix of hydrophilic fibers, such as a web of cellulosic fluff, mixed with particles of high-absorbency material. In particular arrangements, the liquid retention structure 34 may include a mixture of superabsorbent hydrogel-forming particles or fibers and synthetic polymer meltblown fibers, or a mixture of superabsorbent particles or fibers with a fibrous coform material including a blend of natural fibers and/or synthetic polymer fibers.

The hydrophilic fibers and high-absorbency particles can be configured to form an average composite basis weight which may be within the range of about 400 - 900 gsm. In certain aspects of the invention, the average composite basis weight may be within the range of about 500 - 800 gsm, and alternatively may be within the range of about 550 - 750 gsm to provide desired performance. Ultimately, average composite basis weight will depend upon the application to which the personal care garment is put and the type and amount of hydrophilic materials.

Optionally, a substantially hydrophilic tissue wrapsheet may be employed to help maintain the integrity of the fibrous structure of the liquid retention structure 34. The tissue wrapsheet is typically placed about the liquid retention structure over at least the two major facing surfaces thereof and composed of an absorbent cellulosic material, such as creped wadding or a high wet-strength tissue that may or may not be pleated. In one aspect of the invention, the tissue wrapsheet can be configured to provide a wicking layer which helps to rapidly distribute liquid over the mass of absorbent fibers including the liquid retention structure 34. The wrapsheet material on one side of the absorbent fibrous mass may be bonded to the wrapsheet located on the opposite side of the fibrous mass to effectively entrap the liquid retention structure 34.

With reference to Fig. 1, each of the leg elastic members 36 can include a plurality of elastomeric strands. Optionally, each leg elastic member 36 may be a composite that includes at least one carrier layer (not shown), and the elastomeric strands can be operatively attached to the carrier layer. Various mechanisms, such as adhesive, thermal bonds, sonic bonds, or the like as well as combinations thereof, can be employed to provide the desired attachments between the elastomeric strands and the carrier layer. For example, each leg elastic member 36 may be composed of a laminate of a plurality of elastomeric strands sandwiched and held between a pair of carrier layers. The carrier layers may desirably be composed of a woven or nonwoven fabric having a basis weight within the range of about 10-50 g/m², but may optionally be composed of a polymer film material. For example, the carrier layers may be composed of a polypropylene spunbond nonwoven fabric, and the pair of carrier layers may be adhesively bonded together with a suitable pattern of adhesive, such as a swirl-pattern of pressure-sensitive adhesive.

The leg elastic members 36 may have any of a multitude of configurations. For example, the width of the individual elastic members 36 may be varied from about 0.25 millimeters (0.01 inch) to about 25 millimeters (1.0 inch) or more. The elastic members may include a single strand of elastic material, or may include several parallel or non-parallel strands of elastic material, or may be applied in a rectilinear or curvilinear arrangement. Where the strands are non-parallel, two or more of the strands may intersect or otherwise interconnect within the elastic member. The elastic members may be affixed to the backsheet 30 or topsheet 32, or both, of the diaper 20 in any of several ways that are known in the art. For example, the elastic members may be ultrasonically bonded, heat and pressure sealed using a variety of bonding patterns, or adhesively bonded to the diaper with sprayed or swirled patterns of adhesive. In particular embodiments of the invention, the leg elastic members 36 may include a carrier sheet to which are attached a grouped set of elastics composed of a plurality of individual elastic strands. The elastic strands may intersect or be interconnected, or be entirely separated from each other. The carrier sheet may, for example, include a 0,051 mm (0.002 inch) thick polymer film, such as a film of unembossed polypropylene material. The elastic strands can, for example, be composed of LYCRA elastomer available from DuPont, a business having offices in Wilmington, Delaware. Each elastic strand is typically within the range of about 470-1500 decitex (dtx), and may be about 940 - 1050 dtx. In particular embodiments of the invention, for example, three or four strands can be employed for each elasticized leg band.

### Processing Examples

The following examples are presented to provide a more detailed understanding of the invention. The examples are representative, and are not intended to limit the scope of the invention.

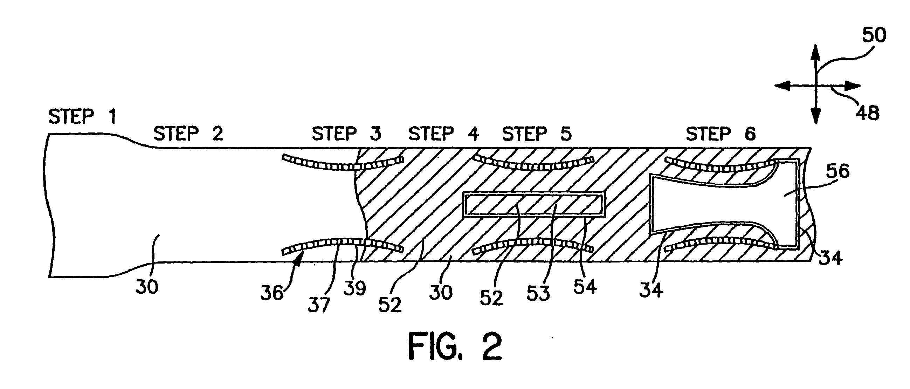

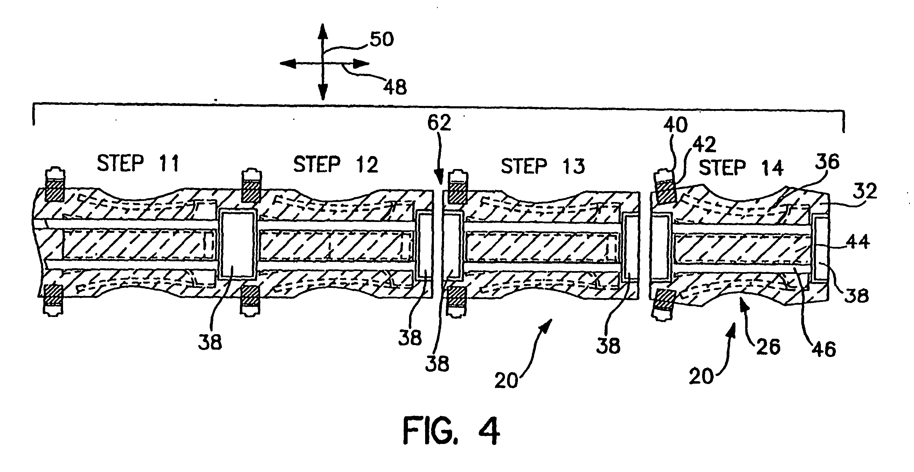

With reference to Figs. 2-4, a method for constructing an absorbent article such as a disposable diaper according to the present invention may include the steps of constructing a precursor garment web and individuating the diapers therefrom as described below and as illustrated in Figs. 2-4.

Referencing Fig. 2, in Step 1 a backsheet web 30 desirably including a web of substantially liquid impermeable and water vapor permeable material, is fed into the garment making process as the foundation layer of the precursor garment web. Backsheet material 30 will extend in the longitudinal direction and retract in the lateral direction when tension is applied in the longitudinal direction, i.e. necking, and can extend in the lateral direction after the tension is removed.

In Step 2 tension is applied to the backsheet 30 which elongates and necks.

In Step 3 a stretched leg elastic members 36, e.g., composed of four LYCRA strands 37 for each leg of Lycra XA Spandex 740 DTEXZ T151 dull (/T-127) from E.I. DuPont de Nemours and Co., of Wilmington, Delaware, and adhesively laminated to a carrier sheet 39, e.g., K-T Slit and Spooled 0,017 mm (0.67 mil) ALE Carrier Sheet from K. T. Industries Inc., of Winnipeg, Manitoba, Canada, are applied to the top of backsheet material web 30.

In Step 4 an adhesive 52, shown as diagonal lines, e.g., Disposamelt 34-5611, from National Starch and Chemical Co., of Bridgewater N.J., is applied to the backsheet material 30.

In Step 5 an adhesive 52, e.g., Disposamelt 34-5611, is applied to the top surface 53 of a spacer layer 54, e.g., a 27,1 gsm (0.8 osy) spunbond-meltblown-spunbond nonwoven web which is then applied to the backsheet material 30.

In Step 6 a liquid retention structure 34, e.g., including a composite fluff pad that is approximately a 60/40 blend of a superabsorbent material e.g., Favor SXM-880, from Stockhausen of Greensboro, NC, and fiberized Fluff Pulp of 16% Hardwood, from Alliance Forest Products of Coosa Pines, AL, is applied to the backsheet material 30. The liquid retention structure 34 may further be covered on its side to be adjacent to topsheet 32 (Step 8) with a barrier tissue 56, e.g., American Tissue 5,68 kg (12.5 pound) white tissue from American Tissue Corp. ofNccnah, WI. Additionally a forming tissue (not shown) e.g., white 4,44 kg (9.79 pound) per reel tissue from American Tissue Corp. of Neenah, WI, may be positioned to cover the side of the liquid retention (absorbent) structure 34 adjacent to the backsheet layer 30.

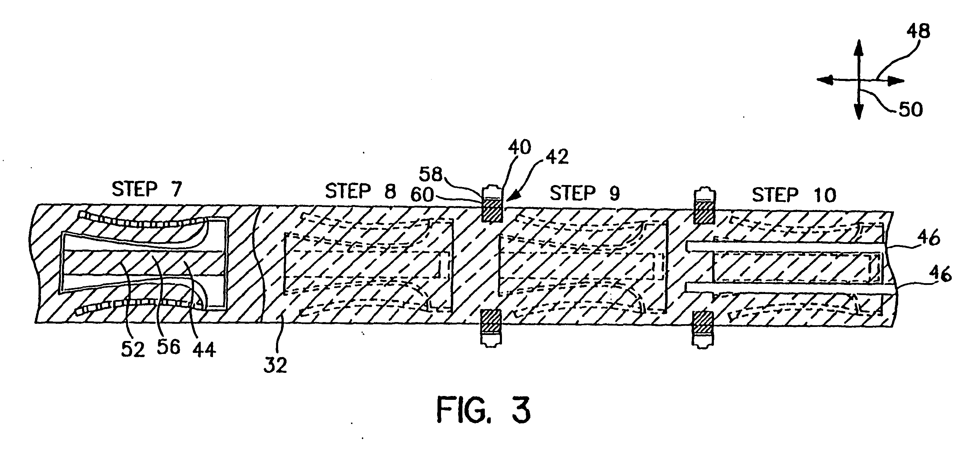

Referencing Fig. 3, in Step 7 a surge management layer 44 e.g., a Through Air Bonded Carded Web nonwoven surge composite for the rapid uptake and channeling of liquids, is located operatively adjacent to the liquid retention structure 34. An adhesive 52, e.g., Disposamelt 34-5611, is applied to the top surface 56 of the surge management layer 44.

In Step 8 a porous, liquid permeable, and necked topsheet web 32, indicated by broken cross hatching, which is laterally extendible when tension is removed in the longitudinal direction 48; is then applied and laminated to the layers of the precursor garment web. Alternately, the topsheet web 32, i.e., the web which will become the topsheet in the finished garment, may have similar properties to the backsheet 30 web as described above.

In Step 9 the provided side panels 42 having a fastening means 40, e.g., hook material such as VELCRO 851 hook, from Velcro USA Inc. of Manchester, NH, and carrier sheet 58, e.g., 42,4 gsm (1.25 osy) spunbond-meltblown-spunbond, and an elastic member 60, e.g., Necked Bonded laminate, such as taught in U.S. Patents 4,981,747 and 5,336,545; may them be adhesively or ultrasonically laminated, or both, to the precursor garment web.

In Step 10 the containment flaps 46 may be adhesively laminated to the precursor garment web and have elastomeric materials, e.g., two elastic strands (not shown) such as Glospan S7 Spandex fiber 700 denier (777 decitex) from Radicapandex (Globe Manufacturing) of Fall River, MA. The flap elastic strands may be laminated to a nonwoven material e.g., blue spunbond-meltblown-spunbond 22.0 gsm (0.65 osy) to comprise the flaps 46.

Referencing Fig, 4, in Step 11 waist elastics 38, e.g., a Necked Bonded laminate may then be applied to the precursor garment web extending across the waistband region of the precursor garments. Alternatively, the waist elastics 38 may be tensioned and then applied to the precursor garment web. Leg hole cut outs on the lateral margins of the precursor diaper will be seen as introduced in this step.

In Step 12, the precursor garment is then cut, as at Ref. No. 62 into individual disposable diapers, collectively 20.

In Step 13, the resultant diaper 20 can then be folded if desired (not shown). Over time the intermediate section 26, or non waistband area of the diaper, which is not held by the waist elastics 38, an expand in the lateral direction 50 as it contracts in the longitudinal direction 48, thus laterally narrowing the waistband area relative to the remaining intermediate area 26 of the diaper 20.

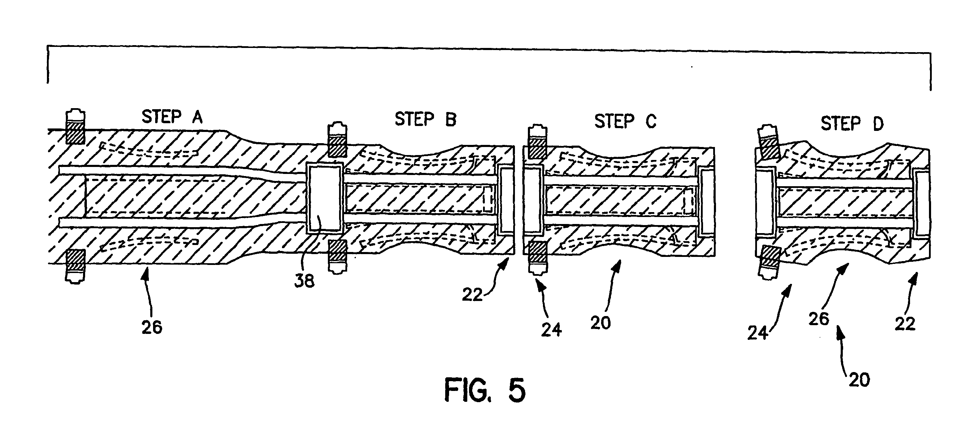

Referencing Fig. 5, an alternative method is illustrated where the diaper assembly is produced with all but the waistband elastic and is then longitudinally stretched in Step A. This stretching causes the entire diaper assembly to try to neck However, stretching and necking may not occur very much in the intermediate section 26 locations with the surge layer, absorbent layer, spacer layers, etc., but will occur predominately in the waistband areas 22, 24 where the topsheet and backsheet are the main components of the precursor diaper. Elastomer 38 is then applied to the waistband area in Step B. A narrowed waistband area is formed after the stretching/tensioning force is removed as the diaper 20 is individuated at Step C and the elastomeric holds the necked waistband areas 22, 24 at the narrower dimension while the intermediate area 26 of the diaper regains its wider dimension at Step D.

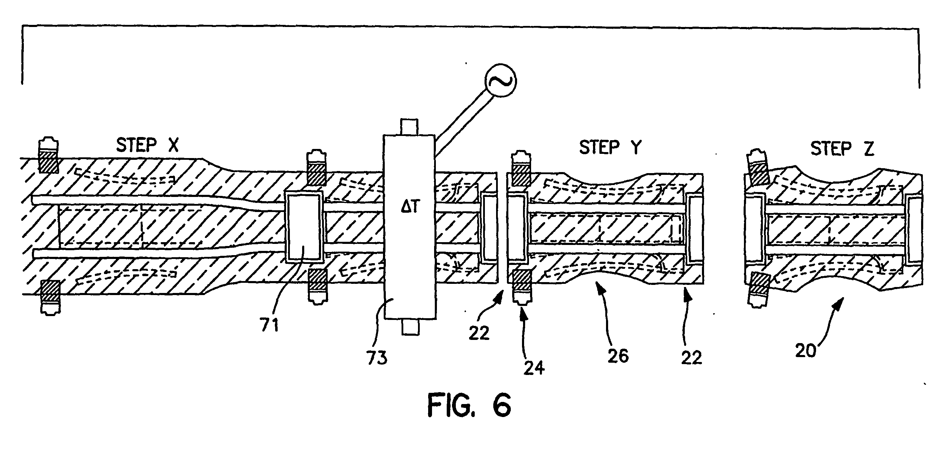

Referencing Fig. 6, an alternative method is illustrated where an unstretched elastomer or precursor elastic 71 can be applied to the precursor diaper web assembly and the assembly/elastomer stretched longitudinally at Step X. Again, the waistband areas 22, 24 should be the areas that neck the most. Heating the elastomer to a high enough temperature, such as with a heated roller 73, while it is necked will cause the elastomer 71 to set or activate at the narrowed width to form the waistband assembly. Narrowed waistband areas 22, 24 are formed after the stretching/tensioning force is removed at Step Y and the elastomeric 71 holds the necked waistband areas at the narrower dimension while the intermediate area 26 of the diaper 20 regains its wider dimension at step Z after individuation of the diaper 20. This method and the method of Fig. 5 could also be used with a thermoset precursor elastic where the elastic precursor is printed on the necked or to-be-necked waistband area and then cross-linked to become elastomeric.

While the invention has been described in detail with respect to specific embodiments thereof, it will be appreciated that those skilled in the art, upon attaining an understanding of the foregoing may readily conceive of alterations to, variations of, and equivalents to these embodiments. Accordingly, the scope of the present invention should be assessed as that of the appended claims and any equivalents thereto.

## Claims

1. A method of producing selectively elastic areas in a web (30) suitable for garment, the web (30) having a longitudinal direction and a lateral direction (50), the method comprising:
a) necking the web (30) to a first width, with width being measured in the lateral direction, the web being expandable to a second non-necked width wider than the first width when the necking tension is removed;
b) affixing an elastic material (3 8) to a selected area (22, 24) of the web when the web is at the first width; and
c) removing necking tension from the web with the elastic (38) thereon and causing the web (30) to assume the second width at areas outside the selected area of the web, and whereby the elastic material holds the selected area of the web at a dimension narrower than the second width, wherein the method is a method of producing elastic cuffs for resultant garments from precursor garments, said web being a precursor garment web, and said selected area being a cuff area.

2. The method according to Claim 1, wherein the step of affixing an elastic material (38) to the cuff area (22, 24) further comprises applying a pre-elastic when the precursor garment web (30) is at the first width, and treating the pre-elastic to become an elastomeric while the precursor garment web is at the first width.

3. The method according to Claim 1 or 2, wherein the precursor garments comprise a backsheet web layer (30).

4. The method according to Claim 3, wherein precursor garments further comprise a topsheet web layer (32).

5. The method according to any of Claims 1 to 4, wherein the precursor garments comprise an assembled diaper lacking only the cuff area.

6. The method according to Claim 3, wherein the backsheet (30) comprises material selected from the group comprising: neckable nonwovens, neckable films, neckable laminates, or combinations thereof.

7. The method according to any of Claims 1 to 6, wherein the elastic material (3 8) is untensioned when applied to the precursor garment web (30), whereby it merely holds the necked material at the first narrower dimension thereby resulting in a flat cuff area.

8. The method according to any of Claims 1 to 6, wherein the elastic material (38) is applied under tension thereby gathering the precursor web (30) and providing a doubly expandable cuff area (22, 24) with a first stage expansion taking out the gathers, and a second stage expansion expanding the material of the garment body.

9. The method according to any of Claims 1 to 6, further including applying a precursor elastic to the cuff area and treating the precursor elastic to become elastomeric while the cuff area is at the first width.

10. The method according to Claim 9, further including treating the precursor elastic with heat.

11. The method according to any of Claims 1 to 10, wherein the cuff area is a leg cuff area.

12. The method according to any of Claims 1 to 10, wherein the cuff area is a waistband area.

13. The method of any preceding claim, wherein said elastic material includes a plurality of elastomeric strands.

## Patentansprüche

1. Verfahren zum Herstellen selektiv elastischer Bereiche in einer Bahn (30), welche für Kleidung geeignet ist, wobei die Bahn (30) eine Longitudinalrichtung und eine Lateralrichtung (50) aufweist, wobei das Verfahren umfasst:
a) Verengen der Bahn (30) zu einer ersten Breite, wobei Breite in der Lateralrichtung gemessen wird, wobei die Bahn zu einer zweiten nicht-verengten Breite dehnbar ist, welche breiter ist als die erste Breite, wenn die Verengungsspannung entfernt wird;
b) Befestigen eines elastischen Materials (38) an einem ausgewählten Bereich (22, 24) der Bahn, wenn die Bahn die erste Breite aufweist; und
c) Entfernen der Verengungsspannung von der Bahn mit der Elastik (38) darauf und Verursachen, dass die Bahn (30) die zweite Breite in Bereichen außerhalb des ausgewählten Bereichs der Bahn einnimmt, und wobei das elastische Material den ausgewählten Bereich der Bahn bei einer Dimension hält, welche enger ist als die zweite Breite, wobei das. Verfahren ein Verfahren zum Herstellen elastischer Bündchen für aus Kleidungsvorläufern resultierende Kleidung ist, wobei die Bahn eine Kleidungsvorläuferbahn ist, und wobei der ausgewählte Bereich ein Bündchenbereich ist.

2. Das Verfahren gemäß Anspruch 1, wobei der Schritt des Befestigens eines elastischen Materials (36) an dem Bündchenbereich (22, 24) des Weiteren das Aufbringen einer Pre-Elastik umfasst, wenn die Kleidungsvorläuferbahn (30) eine erste Breite aufweist, und das Behandeln der Pre-Elastik umfasst, um ein Elastomer zu werden, während die Kleidungsvorläuferbahn die erste Breite aufweist.

3. Das Verfahren gemäß Anspruch 1 oder 2, wobei die Kleidungsvorläufer eine Rückseitenbahnschicht (30) umfassen.

4. Das Verfahren gemäß Anspruch 3, wobei die Kleidungsvorläufer des Weiteren eine Oberseitenbahnschicht (32) umfassen.

5. Das Verfahren gemäß einem der Ansprüche 1 bis 4, wobei die Kleidungsvorläufer eine zusammengesetzte Windel umfassen, welcher nur der Bündchenbereich fehlt.

6. Das Verfahren gemäß Anspruch 3, wobei die Rückseite (30) Material umfasst, welches ausgewählt ist aus der Gruppe umfassend: verengbare Vliese, verengbare Filme, verengbare Laminate oder Kombinationen davon.

7. Das Verfahren gemäß einem der Ansprüche 1 bis 6, wobei das elastische Material (38) ungespannt ist, wenn es auf die Kleidungsvorläuferbahn (30) aufgebracht wird, wobei es das verengte Material lediglich bei einer ersten engeren Dimension hält, dadurch in einem flachen Bündchenbereich resultierend.

8. Das Verfahren gemäß einem der Ansprüche 1 bis 6, wobei das elastische Material (38) unter Spannung aufgebracht wird, dadurch Kräuseln der Vorläuferbahn (30) und Bereitstellen eines doppelt dehnbaren Bündchenbereichs (22, 24) mit einer ersten Dehnungsstufe durch Herausnehmen der Kräuselungen und einer zweiten Dehnungsstufe durch Dehnen des Materials des Kleidungskörpers.

9. Das Verfahren gemäß einem der Ansprüche 1 bis 6, welches des Weiteren das Aufbringen einer Vorläuferelastik auf den Bündchenbereich und das Behandeln der Vorläuferelastik umfasst, um elastomer zu werden, während der Bündchenbereich die erste Breite aufweist.

10. Das Verfahren gemäß Anspruch 9, welches das Weiteren das Behandeln der Vorläuferelastik mit Wärme beinhaltet.

11. Das Verfahren gemäß einem' der Ansprüche 1 bis 10, wobei der Bündchenbereich ein Beinbündchenbereich ist.

12. Das Verfahren gemäß einem der Ansprüche 1 bis 10, wobei der Bündchenbereich ein Bundbereich ist.

13. Das Verfahren gemäß einem der vorherigen Ansprüche, wobei das elastische Material eine Vielzahl an elastomeren Strängen beinhaltet.

## Revendications

1. Procédé de production de zones sélectivement élastiques dans un voile (30) convenant pour des vêtements, le voile (30) ayant une direction longitudinale et une direction transversale (50), le procédé comprenant :
a) le rétrécissement par striction du voile (30) jusqu'à une première largeur, la largeur étant mesurée dans la direction transversale, le voile étant extensible jusqu'à une seconde largeur non sous striction, supérieure à la première largeur, lorsqu'est supprimée la tension de striction ;
b) la fixation d'un matériau élastique (38) à une zone sélectionnée (22,24) du voile lorsque le voile est dans sa première largeur ; et
(c) la suppression de la tension de striction appliquée au voile sur lequel est l'élastique (38) et le fait de causer le retour du voile (30) à la seconde largeur dans des zones extérieures à la zone sélectionnée du voile, grâce à quoi le matériau élastique maintient la zone sélectionnée du voile à une dimension plus étroite que la seconde largeur, ledit procédé étant un procédé de production de tours élastiques de partie corporelle pour des vêtements résultant de précurseurs de vêtement, ledit voile étant un voile de précurseur de vêtement et ladite zone sélectionnée (22,24) étant une zone de tour de partie corporelle.

2. Procédé selon la revendication 1, dans lequel l'étape de fixation d'un matériau élastique (38) à la zone de tour de partie corporelle (22,24) comprend, en outre, l'application d'un pré-élastique tandis que le voile de précurseur de vêtement (30) est à la première largeur, et le traitement du pré-élastique pour qu'il devienne élastomère tandis que le voile de précurseur de vêtement est à la première largeur.

3. Procédé selon la revendication 1 ou 2, dans lequel les précurseurs de vêtement comprennent une couche de voile de feuille dossier (30).

4. Procédé selon la revendication 3, dans lequel les précurseurs de vêtement comprennent, en outre, une couche de voile de feuille supérieure (32).

5. Procédé selon l'une quelconque des revendications 1 à 4, dans lequel les précurseurs de vêtement constituent des couches-culottes assemblées auxquelles ne manque que la zone de tour de partie corporelle.

6. Procédé selon la revendication 3, dans lequel la feuille dossier (30) comprend un matériau sélectionné dans le groupe comprenant : les non-tissés rétrécissables par striction, les films rétrécissables par striction, les stratifiés rétrécissables par striction, et leurs combinaisons.

7. Procédé selon l'une quelconque des revendications 1 à 6, dans lequel le matériau élastique (38) n'est pas sous tension lorsqu'il est appliqué sur le voile de précurseur de vêtement (30), grâce à quoi il se borne à maintenir le matériau rétréci par striction à la première dimension plus étroite, avec pour résultat une zone plate de tour de partie corporelle.

8. Procédé selon l'une quelconque des revendications 1 à 6, dans lequel le matériau élastique (38) est appliqué sous tension, fronçant ainsi le voile de précurseur (30) et réalisant une zone de tour de partie corporelle (22,24) doublement extensible, avec un premier stade d'allongement supprimant les fronces et un second stade d'allongement dilatant le matériau du corps de vêtement.

9. Procédé selon l'une quelconque des revendications 1 à 6, incluant, en outre, l'application d'un précurseur d'élastique à la zone de tour de partie corporelle et le traitement du précurseur d'élastique pour qu'il devienne élastomère tandis que la zone de tour de partie corporelle est à la première largeur.

10. Procédé selon la revendication 9, incluant, en outre, le traitement du précurseur d'élastique par la chaleur.

11. Procédé selon l'une quelconque des revendications 1 à 10, dans lequel la zone de tour de partie corporelle est une zone de tour de jambe.

12. Procédé selon l'une quelconque des revendications 1 à 10, dans lequel la zone de tour de partie corporelle est une zone de ceinture.

13. Procédé selon l'une quelconque des revendications précédentes, dans lequel ledit matériau élastique inclut une pluralité de brins élastomères.
